(19) Europäisches Patentamt

European Patent Office

Office européen des brevets

(11) **EP 1 403 290 A1**

(12) **EUROPEAN PATENT APPLICATION**

(43) Date of publication:
**31.03.2004 Bulletin 2004/14**

(51) Int Cl.⁷: **C08F 8/18**, C07K 1/04

(21) Application number: **03255815.7**

(22) Date of filing: **17.09.2003**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **30.09.2002 US 414762 P**

(71) Applicant: **Rohm and Haas
Philadelphia, Pennsylvania 19106-2399 (US)**

(72) Inventors:
• **Bohling, James Charles
Lansdale Pennsylvania 19446 (US)**

• **Kinzey, Marlin Kenneth
Philadelphia Pennsylvania 19147 (US)**
• **Maikner, John Joseph
Zionsville Pennsylvania 18092 (US)**
• **Zabrodski, William Joseph
Lansdale Pennsylvania 19446 (US)**

(74) Representative: **Kent, Venetia Katherine
Rohm and Haas (UK) Ltd
European Operations Patent Dept.
Lennig House
2 Mason's Avenue
Croydon, CR9 3NB (GB)**

(54) **Resin for solid phase synthesis**

(57) A crosslinked polymer bead which, when: (i) functionalized with a 2-chlorotrityl chloride group; (ii) coupled with Leu to 0.65 mmol/g; and (iii) coupled with Glu(t-Bu); allows coupling of FMOC-Lys(BOC)-OH at an amount of 1.5 equivalents in the presence of 1.5 equivalents of HOBT, 1.5 equivalents of DIEA and 1.5 equivalents of HBTU, to be completed, as determined by the Kaiser test, in no more than 35 minutes.

**Description**

This invention relates to functionalized polymeric resins useful as supports in solid phase synthesis. The present invention also relates to methods to prepare such resins.

Lightly crosslinked resins have found significant utility as solid supports for solid phase organic synthesis processes, such as for the production of polypeptides from amino acids. Such resins typically are less than three percent crosslinked. Accessibility of functional groups on the resin is of great importance in solid phase synthesis, as it controls yields, kinetics, raw material use, and product purity. Lowering the resin crosslinking can increase the ability of the resin to swell, which increases accessibility of functional groups. See, for example, S. Rana et al., "Influence of Resin Cross-Linking on Solid-Phase Chemistry," *J. Comb. Chem*., 2001, 3, 9-15. However, this leads to a softer resin, which is more difficult to process, and to greater volume requirements in the resin bed.

The problem addressed by this invention is to develop a crosslinked polymeric support for solid phase synthesis having accessible functional groups without a high degree of swelling.

**STATEMENT OF INVENTION**

The present invention provides a crosslinked polymer bead which, when: (i) functionalized with a 2-chlorotrityl chloride group; (ii) coupled with Leu to 0.65 mmol/g; and (iii) coupled with Glu(t-Bu); allows coupling of FMOC-Lys (BOC)-OH at an amount of 1.5 equivalents in the presence of 1.5 equivalents of HOBT, 1.5 equivalents of DIEA and 1.5 equivalents of HBTU, to be completed, as determined by the Kaiser test, in no more than 35 minutes.

The present invention further provides a functionalized crosslinked polymer bead produced by a method comprising steps of: (a) swelling the bead in a first solvent or solvent mixture to a volume from 200% to 310% of its volume when dry; and (b) contacting the bead with a functionalizing reagent in a second solvent or solvent mixture capable of swelling the bead to a volume from 200% to 310% of its volume when dry.

The present invention further provides a functionalized crosslinked polymer bead produced by contacting the bead at 100% to 200% of its volume when dry with a functionalizing reagent in a solvent or solvent mixture capable of swelling the bead to a volume from 200% to 400% of its volume when dry.

**DETAILED DESCRIPTION**

Percentages are weight percentages, unless specified otherwise. As used herein the term "(meth)acrylic" refers to acrylic or methacrylic. The term "vinyl monomer" refers to a monomer suitable for addition polymerization and containing a single polymerizable carbon-carbon double bond. The term "styrene polymer" indicates a copolymer polymerized from a vinyl monomer or mixture of vinyl monomers containing at least 50 weight percent, based on the total monomer weight, of styrene monomer, along with at least one crosslinker. Preferably a styrene polymer is made from a mixture of monomers that is at least 75% styrene, more preferably at least 90% styrene, and most preferably from a mixture of monomers that consists essentially of styrene and at least one vinylaromatic crosslinker. The polymeric bead used as a starting material in this invention contains monomer residues from at least one monomer having one copolymerizable carbon-carbon double bond and at least one crosslinker. The monomer residues derived from the crosslinker are from 0.5 mole percent to 1.5 mole percent based on the total of all monomer residues. Preferably the amount of crosslinker is from 0.7 to 1.3 mole percent, more preferably from 0.7 to 1.2 mole percent, and most preferably from 0.8 to 1.2 mole percent.

A polymeric bead used as a starting material in the present invention preferably is a spherical copolymer bead having a particle diameter no greater than 200 microns (μm), preferably no greater than 170 μm, more preferably no greater than 150 μm, more preferably no greater than 125 μm, and most preferably no greater than 100 μm. Preferably, the bead has no void spaces having a diameter greater than 3 μm, more preferably no void spaces having a diameter greater than 2 μm, and most preferably no void spaces having a diameter greater than 1 μm. Typically, void spaces are readily apparent upon surface examination of the bead by a technique such as light microscopy.

The polymeric bead used as a starting material in the present invention preferably is produced by a suspension polymerization. A typical bead preparation, for example, may include preparation of a continuous aqueous phase solution containing typical suspension aids, for example, dispersants, protective colloids and buffers. Preferably, to aid in production of relatively small beads, a surfactant is included in the aqueous solution, preferably a sodium alkyl sulfate surfactant, and vigorous agitation is maintained during the polymerization process. The aqueous solution is combined with a monomer mixture containing at least one vinyl monomer, at least one crosslinker and at least one free-radical initiator. Preferably, the total initiator level is from 0.25 mole percent to 2 mole %, based on the total monomer charge, preferably from 0.4 mole percent to 1.5 mole percent, more preferably from 0.4 mole percent to 1 mole percent, and most preferably from 0.5 mole percent to 0.8 mole percent. The mixture of monomers is then polymerized at elevated temperature. Preferably, the polymerization is continued for a time sufficient to reduce the unreacted vinyl monomer content to less than 1% of the starting amount. The resulting bead is then isolated by conventional means, such as dewatering, washing with an aprotic organic solvent, and drying.

Crosslinkers are monomers having 2 or more copolymerizable carbon-carbon double bonds per molecule, such as: divinylbenzene, divinyltoluene, divinylxylene, trivinylbenzene, trivinylcyclohexane, divinylnaphthalene, trivinylnaphthalene, diethyleneglycol divinylether, ethyleneglycol dimethacrylate, polyethyleneglycol dimethacrylate, triethyleneglycol dimethacrylate, trimethylolpropane trimethacrylate, allyl methacrylate, 1,5-hexadiene, 1,7-octadiene or 1,4-bis(4-vinylphenoxy)butane; it is understood that any of the various positional isomers of each of the aforementioned crosslinkers is suitable. Preferred crosslinkers are divinylbenzene, divinyltoluene, trivinylbenzene or 1,4-bis(4-vinylphenoxy)butane. The most preferred crosslinker is divinylbenzene.

Suitable monounsaturated vinylaromatic monomers that may be used in the preparation of the bead used as a starting material in the present invention include, for example, styrene, $\alpha$-methylstyrene, $(C_1-C_4)$alkyl-substituted styrenes and vinylnaphthalene; preferably one or more monounsaturated vinylaromatic monomer is selected from the group consisting of styrene and $(C_1-C_4)$alkyl-substituted styrenes. Included among the suitable $(C_1-C_4)$alkyl-substituted styrenes are, for example, ethylvinylbenzenes, vinyltoluenes, diethylstyrenes, ethylmethylstyrenes, dimethylstyrenes and isomers of vinylbenzyl chloride; it is understood that any of the various positional isomers of each of the aforementioned vinylaromatic monomers is suitable.

Optionally, non-aromatic vinyl monomers, such as aliphatic unsaturated monomers, for example, acrylonitrile, glycidyl methacrylate, (meth)acrylic acids and amides or $C_1-C_6$ alkyl esters of (meth)acrylic acids may also be used in addition to the vinylaromatic monomer. When used, the non-aromatic vinyl monomers typically comprise as polymerized units, from zero to 20%, preferably from zero to 10%, and more preferably from zero to 5% of the copolymer, based on the total monomer weight used to form the copolymer.

Preferred vinyl monomers are the vinylaromatic monomers; more preferably styrene, isomers of vinylbenzyl chloride, and $\alpha$-methylstyrene. The most preferred vinyl monomer is styrene.

Polymerization initiators useful in the present invention include monomer-soluble initiators such as peroxides, hydroperoxides, peroxyesters and related initiators; for example benzoyl peroxide, *tert*-butyl hydroperoxide, cumene peroxide, tetralin peroxide, acetyl peroxide, caproyl peroxide, *tert*-butyl peroctoate (also known as *tert*-butylperoxy-2-ethylhexanoate), *tert*-amyl peroctoate, *tert*-butyl perbenzoate, *tert*-butyl diperphthalate, dicyclohexyl peroxydicarbonate, di(4-*tert*-butylcyclohexyl)peroxydicarbonate and methyl ethyl ketone peroxide. Also useful are azo initiators such as azodiisobutyronitrile, azodiisobutyramide, 2,2'-azo-*bis*(2,4-dimethylvaleronitrile), azo-*bis*($\alpha$-methyl-butyronitrile) and dimethyl-, diethyl- or dibutyl azo-*bis*(methylvalerate). Preferred peroxide initiators are diacyl peroxides, such as benzoyl peroxide, and peroxyesters, such as *tert*-butyl peroctoate and *tert*-butyl perbenzoate.

Dispersants and suspending agents useful in the present invention are nonionic surfactants having a hydroxyalkylcellulose backbone, a hydrophobic alkyl side chain containing from 1 to 24 carbon atoms, and an average of from 1 to 8, preferably from 1 to 5, ethylene oxide groups substituting each repeating unit of the hydroxyalkyl-cellulose backbone, the alkyl side chains being present at a level of 0.1 to 10 alkyl groups per 100 repeating units in the hydroxyalkylcellulose backbone. The alkyl group in the hydroxyalkylcellulose may contain from 1 to 24 carbons, and may be linear, branched or cyclic. More preferred is a hydroxyethylcellulose containing from 0.1 to 10 $(C_{16})$alkyl side chains per 100 anhydroglucose units and from about 2.5 to 4 ethylene oxide groups substituting each anhydroglucose unit. Typical use levels of dispersants are from about 0.01 to about 4%, based upon the total aqueous-phase weight.

Optionally, the preparation of the beads may include an enzyme treatment to cleanse the polymer surface of residues of dispersants and suspending agents used during the polymerization. The enzyme treatment typically involves contacting the polymeric phase with the enzymatic material (selected from one or more of cellulose-decomposing enzyme and proteolytic enzyme) during polymerization, following polymerization or after isolation of the polymer. Japanese Patent Applications No. 61-141704 and No. 57-98504 may be consulted for further general and specific details on the use of enzymes during the preparation of polymer resins. Suitable enzymes include, for example, cellulose-decomposing enzymes, such as $\beta$-1,4-glucan-4-glucano-hydrase, $\beta$-1,4-glucan-4-glucanhydrolase, $\beta$-1,4-glucan-4-glucohydrase and $\beta$-1,4- glucan-4-cellobiohydrase, for cellulose-based dispersant systems; and proteolytic enzymes, such as urokinase, elastase and enterokinase, for gelatin-based dispersant systems. Typically, the amount of enzyme used relative to the polymer is from 2 to 35%, preferably from 5 to 25% and more preferably from 10 to 20%, based on total weight of polymer.

In the method of the present invention, the swelling of the crosslinked polymeric beads is controlled so that the bead is partially swelled during functionalization. Without wishing to be bound by theory, the effect of functionalizing a partially swollen bead is to limit the location of the attached functional groups to a region relatively close to the surface of the bead. Preferably, when the functionalization occurs, the bead is swollen to at least 200% of its volume when dry, more preferably at least 210%, more preferably at least 220%, more preferably at least 230%, and most preferably at least 240%. Preferably, the bead is swollen to no more than 310% of its volume when dry, more preferably no more than 300%, more preferably no more than 290%, and most preferably no more than 280%. There are different means for accomplishing the desired degree of swelling during functionalization.

In one embodiment of the invention, a bead which is not pre-swollen (i.e., at 100% of its volume when dry), or which is pre-swollen to no more than 200% of its volume when dry, is contacted with a functionalizing reagent in a solvent or solvent mixture capable of swelling the bead to at least 200% of its volume when dry, more preferably at least 210%, more preferably at least 220%, more preferably at least 230%, and most preferably at least 240%. Preferably, the solvent or solvent mixture is capable of swelling the bead to no more than 400% of its volume when dry, more preferably no more than 370%, more preferably no more than 340%, and most preferably no more than 320%. Preferably, the bead is pre-swollen to no more than 150%, more preferably no more than 100%, more preferably no more than 80%, more preferably no more than 60%, and most preferably no more than 40%. In one embodiment, the bead is used in its dry state without pre-swelling.

In another embodiment of the invention, the bead is pre-swollen in a solvent or solvent mixture which swells the bead to at least 200% of its volume when dry, more preferably at least 210%, more preferably at least 220%, more preferably at least 230%, and most preferably at least 240%. Preferably, the bead is swollen to no more than 310% of its volume when dry, more preferably no more than 300%, more preferably no more than 290%, and most preferably no more than 280%. After pre-swelling, the bead is contacted with a functionalizing reagent in a solvent or solvent mixture capable of swelling the bead within the aforementioned limits. Most preferably, the solvents or solvent mixtures used for pre-swelling and functionalization are the same.

A functionalizing reagent is one which covalently attaches a functional group to the polymer comprising the bead. Further elaboration of the functional group may be necessary to maximize the utility of the bead as a support for solid phase synthesis. However, the initial attachment of the functional group determines the region of the bead which is functionalized and thus tends to control the ability of the bead to react with substrates for solid phase synthesis and to allow recovery of the synthetic product. For styrene polymers, the functionalization typically is a Friedel-Crafts substitution on the aromatic ring, preferably an acylation, bromination, or halomethylation. Subsequent elaboration of the initial functional group typically is done. For example, acylation by aroyl halides often is followed by addition of an aryl lithium to the carbonyl group of the product to produce a triaryl carbinol functional group, which then is halogenated to produce a trityl halide functional group. In one preferred embodiment of the invention, 2-chlorobenzoyl chloride, followed by phenyllithium, and then thionyl chloride, produces a 2-chlorotrityl chloride functional group. Bromination typically is followed by treatment with an alkyl lithium reagent and reaction of the aryl lithium product with a variety of reagents to produce different functional groups. Halomethyl groups also may react with a variety of reagents to produce different functional groups.

Solvents capable of partially swelling the bead include, for example, $C_1$-$C_6$ nitroalkanes, and mixtures of relatively non-swelling solvents such as alkanes with nitrobenzene or chlorinated hydrocarbons. For functionalization using Friedel-Crafts chemistry, $C_3$-$C_6$ nitroalkanes, and mixtures of relatively non-swelling solvents such as alkanes with nitrobenzene are preferred.

The surface-functionalized beads described herein are useful, for example, in solid-phase organic synthesis, solid-phase peptide synthesis, and scavenging of reaction byproducts. Typically, coupling reactions between the surface-functionalized beads and reagents in solution occur faster than with conventional functionalized polymer beads. For example, when a 2-chlorotrityl-chloride functional group on a surface-functionalized bead described herein reacts with a given concentration of a protected amino acid reagent in the presence of typical coupling reagents used in peptide synthesis, the reaction typically is complete in the same time or a shorter time than that observed for a conventional functionalized bead, as demonstrated below in Example 8 and Table 4. Coupling efficiency for reaction of the surface functionalized bead of this invention with a protected amino acid residue is greater than that of conventional beads, as demonstrated below by weight gain of the beads in Example 6 and Table 2, and by HPLC measurements of cleaved amino acid in Example 7 and Table 3.

Typical loading of amino acid, with or without typical protecting groups well known in peptide synthesis, onto the surface-functionalized beads of this invention is from 0.2 meq/g to 1.0 meq/g, based on the weight of the unloaded beads. In one embodiment of the invention, preferably, at least 0.25 meq/g is loaded, more preferably at least 0.3 meq/g, more preferably at least 0.5 meq/g, and most preferably at least 0.6 meq/g. Preferably, the loading is no more than 0.9 meq/g, more preferably no more than 0.8 meq/g, and most preferably no more than 0.7 meq/g. In another embodiment of the invention, preferably, at least 0.6 meq/g is loaded, more preferably at least 0.7 meq/g, more preferably at least 0.8 meq/g, and most preferably at least 0.9 meq/g. Preferably, the loading is no more than 1.2 meq/g, more preferably no more than 1.1 meq/g, and most preferably no more than 1.0 meq/g.

**EXAMPLES**

Comparative Example 1: Internal and Surface Functionalization of Pre-Swelled Crosslinked Polystyrene Beads

A 1L round bottom flask fitted with an overhead stirrer, $N_2$ inlet fitted with a pressure relief upstream, and a thermocouple was purged with a light positive pressure of nitrogen (sweep against open stopper while making additions). Nitrobenzene (400 mL) was charged and held at room temperature. A polystyrene resin (40 g, 0.379 mol) was charged against the nitrogen sweep and stirred for 1/2 hour. Chlorobenzoyl chloride (24.89 g, 0.142 mol) was charged to the flask and stirred for 15 minutes. Inside a glove bag filled with nitrogen, aluminum chloride (18.96 g, 0.142 mol) was weighed into a sealed bottle, which then was charged into the reaction flask against the nitrogen sweep. The contents of the flask were heated to 30°C and held for 4 hours. The reaction mixture was poured into a buchner filter funnel, and the reaction flask washed with a small amount of nitrobenzene to complete transfer. The filter was drained to resin level, and nitrobenzene (280 mL, 1 bed volume) was added, and the filter drained again. Tetrahydrofuran ("THF") (2 bed volumes) was added on top of resin bed, which was allowed to drain. The color was removed as the THF replaced the nitrobenzene. One bed volume of 4:1 THF:$H_2O$ was added and the resin was re-suspended, then the filter was drained to the resin level and one bed volume of THF was added on top of the resin. The filter was allowed to drain to the resin level. One bed volume of THF was added and the resin was re-suspended, then the filter was drained to the resin level and one bed volume of THF was added on top of the resin. The filter was allowed to drain to the resin level. One bed volume of methanol was added on top of resin. The filter was allowed to drain to the resin level. One bed volume of methanol was added and the resin was re-suspended, then the filter was drained to the resin level and one bed volume of methanol was added on top of the resin. The filter was allowed to drain to the resin level. Minimal vacuum was applied to remove excess solvent. The resin was dried in a 35°C vacuum oven to a constant weight.

Example 1: Surface Functionalization of a Crosslinked Polystyrene Bead by Functionalization of Unswelled Beads

A 1L round bottom flask fitted with an overhead stirrer, $N_2$ inlet fitted with a pressure relief upstream, and a thermocouple was purged with a light positive pressure of nitrogen (sweep against open stopper while making additions). Nitrobenzene (400 mL) was charged and held at room temperature. Inside a glove bag filled with nitrogen, aluminum chloride (18.96 g, 0.142 mol) was weighed into a sealed bottle, which then was charged into the reaction flask against the nitrogen sweep. After the aluminum chloride was dissolved (ca. 5 minutes), chlorobenzoyl chloride (24.89 g, 0.142 mol) was charged to the flask and stirred for 5 minutes. A polystyrene resin (40 g, 0.379 mol) was charged against the nitrogen sweep and stirred for 1/2 hour. The contents of the flask were heated to 30°C and held for an additional 3.5 hours. The reaction mixture was poured into a buchner filter funnel, and the reaction flask washed with a small amount of nitrobenzene to complete transfer. The filter was drained to resin level, and nitrobenzene (280 mL, 1 bed volume) was added, and the filter drained again. Tetrahydrofuran ("THF") (2 bed volumes) was added on top of resin bed, which was allowed to drain. The color was removed as the THF replaced the nitrobenzene. One bed volume of 4:1 THF:$H_2O$ was added and the resin was re-suspended, then the filter was drained to the resin level and one bed volume of THF was added on top of the resin. The filter was allowed to drain to the resin level. One bed volume of THF was added and the resin was re-suspended, then the filter was drained to the resin level and one bed volume of THF was added on top of the resin. The filter was allowed to drain to the resin level. One bed volume of methanol was added on top of the resin. The filter was allowed to drain to the resin level. One bed volume of methanol was added and the resin was re-suspended, then the filter was drained to the resin level and one bed volume of methanol was added on top of the resin. The filter was allowed to drain to the resin level. Minimal vacuum was applied to remove excess solvent. The resin was dried in a 35°C vacuum oven to a constant weight.

Example 2: Surface Functionalization of Crosslinked Polystyrene Beads by Selection of Functionalization Solvent

A 1L round bottom flask fitted with an overhead stirrer, $N_2$ inlet fitted with a pressure relief upstream, and a thermocouple is purged with a light positive pressure of nitrogen (sweep against open stopper while making additions). Nitroethane (400 mL) is charged and held at room temperature. A polystyrene resin (40 g, 0.379 mol) is charged against the nitrogen sweep and stirred for 1/2 hour. Chlorobenzoyl chloride (24.89 g, 0.142 mol) is charged to the flask and stirred for 15 minutes. Inside a glove bag filled with nitrogen, aluminum chloride (18.96 g, 0.142 mol) is weighed into a sealed bottle, which then is charged into the reaction flask against the nitrogen sweep. The contents of the flask are heated to 30°C and held for an additional 3.75 hours. The reaction mixture is poured into a buchner filter funnel, and the reaction flask washed with a small amount of nitrobenzene to complete transfer. The filter is drained to resin level, and nitrobenzene (280 mL, 1 bed volume) is added, and the filter drained again. Tetrahydrofuran ("THF") (2 bed volumes) is added on top of the resin bed, which is allowed to drain. The color is removed as the THF replaces the nitrobenzene. One bed volume of 4:1 THF:$H_2O$ is added and the resin is re-suspended, then the filter is drained to the resin level and one bed volume of THF is added on top of the resin. The filter is allowed to drain to the resin level. One bed volume of THF is added and the resin is re-suspended, then the filter is drained to the resin level and one bed volume of THF is added on top of the resin. The filter is allowed to drain to the resin level. One bed volume of methanol is added on top of resin. The filter is allowed to drain to the resin level. One bed volume of methanol is added and the resin is re-suspended, then the filter is drained to the resin level and one bed volume of methanol is added on top of the resin. The filter is allowed to drain to the resin level. Minimal vacuum is applied to remove excess solvent. The resin is dried in a 35°C vacuum oven to a constant weight.

Example 3: Surface Functionalization of Crosslinked Polystyrene Beads by Use of a Mixed Functionalization Solvent

A 1L round bottom flask fitted with an overhead stirrer, $N_2$ inlet fitted with a pressure relief upstream, and a thermocouple is purged with a light positive pressure of nitrogen (sweep against open stopper while making additions). Nitrobenzene (60 mL) and Heptane (440 mL) are charged and held at room temperature. A polystyrene resin (40 g, 0.379 mol) is charged against the nitrogen sweep and stirred for 1/2 hour. Chlorobenzoyl chloride (24.89 g, 0.142 mol) is charged to the flask and stirred for 15 minutes. Inside a glove bag filled with nitrogen, aluminum chloride (18.96 g, 0.142 mol) is weighed into a sealed bottle, which then is charged into the reaction flask against the nitrogen sweep. The contents of the flask are heated to 30°C and held for 4 hours. The reaction mixture is poured into a buchner filter funnel, and the reaction flask washed with a small amount of nitrobenzene to complete transfer. The filter is drained to resin level, and nitrobenzene (280 mL, 1 bed volume) is added, and the filter drained again. Tetrahydrofuran ("THF") (2 bed volumes) is added on top of resin bed, which is allowed to drain. The color is removed as the THF replaces the nitrobenzene. One bed volume of 4:1 THF:$H_2O$ is added and the resin is re-suspended, then the filter is drained to the resin level and one bed volume of THF is added on top of the resin. The filter is allowed to drain to the resin level. One bed volume of THF is added and the resin is re-suspended, then the filter is drained to the resin level and one bed volume of THF is added on top of the resin. The filter is allowed to drain to the resin level. One bed volume of methanol is added on top of resin. The filter is allowed to drain to the resin level. One bed volume of methanol is added and the resin is re-suspended, then the filter is drained to the resin level and one bed volume of methanol is added on top of the resin. The filter is allowed to drain to the resin level. Minimal vacuum is applied to remove excess solvent. The resin is dried in a 35°C vacuum oven to a constant weight.

Example 4: General Procedure for Final Functionalization of Crosslinked Beads

In an oven dried four neck round bottom flask (equipped with a stirrer, a condenser w/nitrogen bubbler, a temperature controller, and a septum) was taken the THF and the dried bead resulting from any of the previous Examples (10:1, volume:weight). The mixture was stirred for 15 minutes. Phenyl lithium (1.25 equivalents) was added drop wise over 10 minutes. The temperature was kept <30°C by an ice/water bath. The reaction mixture was then stirred at ambient temperature for 1 hour. Quenching was accomplished by drop wise addition of 10% aqueous HCl, keeping the reaction temperature below 30°C. The mixture was stirred for 1 hour. The contents are then transferred to a sinter glass funnel and drained to bed height. The resin was then re-suspended in 1 bed volume of 4:1 THF/10%HCl(v/v) and allowed to drain to bed height slowly. The resin was re-suspended with 1 bed volume of 4:1 THF/water and allowed to drain. The bed was then re-suspended and drained 3 times with 1 bed volume of THF, followed by re-suspending/draining 3 times with 1 bed volume of methanol. A final rinse through of the bed is done with 1 bed volume of methanol. Vacuum was applied to remove excess solvent and then the beads were dried in a 35°C vacuum oven.

In an oven dried four neck round bottom flask (equipped with a stirrer, a temperature controller, a condenser w/nitrogen bubbler, and a stopper) was added the methylene chloride and the dried bead from the previous step (10:1). Added thionyl chloride (5 equivalents) drop-wise followed by N,N-dimethylformamide (5 mole % based on thionyl chloride). The mixture was warmed to reflux (37°C) for 4 hours. After cooling to ambient temperature, the reaction mixture was transferred to a nitrogen purged sintered glass funnel and drained to bed height. The bed was then re-suspended and drained twice with 1 bed volume of methylene chloride. It was then further washed by re-suspending/draining three times with 1 bed volume of anhydrous hexane. Purged through the bed with nitrogen to remove excess solvent and then placed the beads in a vacuum oven at ambient temperature. The trityl chloride functionalized bead resulting from this preparation is useful, for example, in solid phase peptide synthesis.

Example 5: Swelling of Crosslinked Polystyrene Beads in Various Solvents

Crosslinked polystyrene beads made using 1% and approximately 1.5% divinylbenzene as a crosslinker, and having a volume when dry of 1.65 mL/g were swelled in solvents, with the results presented below in Table 1 in mL/g. Solvent ratios are volume:volume.

Table 1

| Solvent | 1.5% crosslinker | 1% crosslinker |
|---|---|---|
| nitromethane | 2.5 | N/A |
| nitropropane | 3.7 | 4.05 |
| 1:1, nitropropane:heptane | 3.6 | 4.3 |
| 1:2, nitropropane:heptane | 3.5 | 3.7 |
| 1:3, nitropropane:heptane | 3.3 | 3.55 |
| nitrobenzene | 4.0 | 5.3 |
| 1:1, nitrobenzene:heptane | 4.6 | 5.6 |
| 1:2, nitrobenzene:heptane | 4.5 | 5.05 |
| 1:3, nitrobenzene:heptane | 4.2 | 4.3 |
| methanol | 1.7 | N/A |
| heptane | 1.9 | N/A |

Example 6: Loading of Surface-Functionalized Crosslinked Beads with Fmoc-L-Leucine

A 2-chlorotrityl chloride resin produced according to Example 4 was loaded with Fmoc-L-Leucine, treated with methanol to remove residual reactive chloride and dried. The weight gain was used to quantify loading. The resin was assumed to have a capacity of 1.3 mmol/g. The relatively minor molecular weight effect of the methoxy end-capping was ignored. The resin was cleaved with 1% TFA/DCM, and the solution was analyzed by HPLC to determine the cleaved yield (recovery) of amino acid.

Each sample of the resin (1.0000 +/- 0.05 g) was weighed into a 60 mL glass synthesizer vessel with a side port and a removable disk. The resin in the synthesizer was pre-swelled with dichloromethane (DCM). The DCM was drained and to each synthesizer was added a solution of Fmoc-L-Leu-OH and diisopropylethylamine (DIEA) in 10 ml DCM. Slow nitrogen agitation was started. For the five resins of this invention, the quantities, in grams, of Fmoc-L-Leu-OH were (3.181, 0.597, 0.358, 0.299, 0.239) and of DIEA, in mL, were (1.568, 0.294, 0.177, 0.147, 0.118) per sample, respectively. Each mixture was allowed to react at ambient temperature for two hours, then the solution was drained and any remaining trityl chloride end groups were capped by treatment for at least 30 minutes with DIEA (1 mL) in methanol (9 mL). Each sample of resin was washed with 5 x 10 mL portions of DCM and transferred to a tared 30 mL fritted glass funnel, then washed with another 2 x 10 mL portions of DCM. Each loaded resin was then de-swelled with 4 x 10mL portions of isopropanol (IPA) and partially dried by pulling air through the filter cake with vacuum, then drying the filter and resin overnight in a vacuum oven at 30°C. The filter and resin were then re-weighed and the difference in mass calculated. Mass of Leu = Final wt-(filter tare+1.000g resin). Loading efficiency = (weight of Leu on resin/weight of Leu charged)* 100. The weight gain and loading efficiency are reported in Table 2 for five resins of this invention (RH1-RH5) and for three competitive resins processed according to the procedures given in this Example (CM1-CM3). The cleaved yield for the same resins is reported in Table 3. The amount of amino acid (AA) is in mmol, the weight gain (gain) in mmol, and the loading efficiency (eff.) in %.

Table 2:

| Weight Gain and Loading Efficiency Comparison | | | | | | | | |
|---|---|---|---|---|---|---|---|---|
| amount of AA | 0.61 | 0.68 | 0.73 | 0.84-0.85 | 0.97 | 1.01 | 1.05 | 1.26 |
| gain, RH1 | | 0.54 | | 0.81 | | 0.97 | | |
| gain, RH2 | | 0.31 | | 0.52 | | 0.79 | | |
| gain, RH3 | | 0.49 | | 0.60 | | 0.82 | | |
| gain, RH4 | | 0.66 | | 0.75 | | 0.98 | | |
| gain, RH5 | | 0.63 | | 0.82 | | 0.86 | | |
| avg. gain, RH1-RH5 | | 0.53 | | 0.70 | | 0.88 | | |
| gain, CM1 | | 0.34 | | 0.53 | | 0.81 | | |

Table 2:   (continued)

| Weight Gain and Loading Efficiency Comparison | | | | | | | | |
|---|---|---|---|---|---|---|---|---|
| amount of AA | 0.61 | 0.68 | 0.73 | 0.84-0.85 | 0.97 | 1.01 | 1.05 | 1.26 |
| gain, CM2 | 0.26 | | 0.32 | | 0.73 | | | |
| gain, CM3 | | | | 0.46 | | | 0.80 | 0.88 |
| eff., RH1 | | 80.5 | | 95.8 | | 96.1 | | |
| eff., RH2 | | 45.5 | | 61.4 | | 77.7 | | |
| eff., RH3 | | 72.5 | | 70.9 | | 80.8 | | |
| eff., RH4 | | 97.7 | | 88.5 | | 97.1 | | |
| eff., RH5 | | 93.2 | | 96.8 | | 84.9 | | |
| avg. eff., RH1-RH5 | | 77.9 | | 82.7 | | 87.3 | | |
| eff., CM1 | | 49.9 | | 62.4 | | 79.8 | | |
| eff., CM2 | 42.5 | | 44.4 | | 75.2 | | | |
| eff., CM3 | | | | 55.2 | | | 76.4 | 70.0 |

CM2 is a resin available from Novabiochem under the name 2-Chlorotritylchloride Resin (100-200 mesh), 1% DVB; Cat. No. 01-64-0114

CM3 is a resin available from Polymer Labs under the name Cl-Trt-Cl Resin (75-150 micron), 1% DVB, Part No. 3473-2799

Example 7: Cleavage of Fmoc-L-Leucine from Surface-Functionalized Beads

Each sample of the resin (1.0000 +/- 0.05 g) was weighed into a fritted glass filter. The resin was pre-swelled by agitating the funnel with DCM (10 mL), the DCM was drained, and the resin washed 3 x 10 mL DCM. The resin bound Fmoc-L-Leu-OH was cleaved by agitating with 9x 10 ml of 1% TFA/DCM (v:v), draining into a 100 mL volumetric flask, and filling to the mark with DCM. The contents of the flask were agitated to provide the sample solution to be analyzed by HPLC.

The sample solution was injected into a liquid chromatographic system capable of generating a binary solvent gradient, and equipped with a sample injector, a variable wavelength detector and electronic data acquisition system (HP 1090 with ChemStation™ software). Column: YMC ODS-AQ, S-3 , 120 A, 50 mm X 4 mm ID column Catalog # AQ12S030504WT

Conditions:

Flow rate:      1.5 mls/min
Program:        40% B, hold 10.0 min
                40 % B to 90% B over 2.5 minutes, hold 1 minute
                90% B over 2 minutes hold 10 minutes until next
                injection.
Injection vol.:   10uL

Detection: photodiode array detector 265 nm bandwidth 16 nm, ref: 350 nm, bw 100 nm, or variable wavelength UV Detector at 265 nm.

Standard preparation:

Approximately 5.0 mg of the Fmoc-L-Leu-OH reference standard were weighed into a 25 mL volumetric flask. The standard was dissolved in about 10 mL of acetonitrile (often requires sonication). Water (12 mL) was added, the contents were mixed, and the flask was allowed to come to ambient temperature. The flask was filled to the mark with water and the contents were mixed.

Sample preparation:

The sample solution (5.00 mL) was measured into a 25 mL volumetric flask, and reduced to dryness at ambient temperature with a gentle nitrogen stream. The residue was dissolved in 10 mL of acetonitrile (often requires sonication). Water (12 mL) was added, the contents were mixed well and the flask allowed to come to ambient temperature. The flask was filled to the mark with water and agitated.
A blank (water: acetonitrile 3:2) was injected and the gradient program started. Concomitantly, the sample and standard were injected.

The loading of the Fmoc-L-Leu on the resin was calculated by:

Fmoc-L-Leu in sample flask = Area sample/Area standard X Wt of standard x Purity of standard/ 25.0 mL X 25.0 mL/ 5.0 mL

Resin Loading (mmol/g of dry resin) =Amt of Fmoc L-Leu-OH in sample flask/ Wt of loaded resin g X 353.4 [353.4 is the molecular weight of the Fmoc-L-Leu-OH]

Results for the amount of cleaved Fmoc L-Leu-OH in mmol ("AA") for each amount of amino acid used to load the resins initially for the RH1 to RH5 and CM1 materials are reported in Table 3. Load efficiencies are also reported, assuming that the cleaved amount equals the amount bound to the resin.

Table 3:

| Cleaved Amino Acid Yield Comparison | | | |
|---|---|---|---|
| amount of AA | 0.68 | 0.85 | 1.01 |
| AA, RH1 | 0.56 | 0.85 | 0.99 |
| AA, RH2 | 0.31 | 0.51 | 0.82 |
| AA, RH3 | 0.50 | 0.61 | 0.84 |
| AA, RH4 | 0.63 | 0.72 | 0.99 |
| AA, RH5 | 0.64 | 0.87 | 0.88 |
| avg. AA, RH1-RH5 | 0.53 | 0.71 | 0.90 |
| AA, CM1 | 0.33 | 0.60 | 0.85 |
| eff., RH1 | 82.4 | 100.0 | 98.0 |
| eff., RH2 | 45.6 | 60.0 | 81.2 |
| eff., RH3 | 73.5 | 71.8 | 83.2 |
| eff., RH4 | 92.6 | 84.7 | 98.0 |
| eff., RH5 | 94.1 | 102.4 | 87.1 |
| avg. eff., RH1-RH5 | 77.6 | 83.8 | 89.5 |
| eff., CM1 | 48.5 | 70.6 | 84.2 |

Example 8: Peptide Build Kinetic Efficiency Comparison

This Example describes the preparation of a nine-peptide fragment of the peptide known as T-20, described in U.S. Pat. No. 6,015,881, Table 1, as Peptide No. 11, containing amino acids 17-26. The kinetics of the reaction are followed by sampling resin periodically during the coupling and running a Kaiser test to determine the presence of any unreacted primary amine. The resin according to Example 4 is compared with two competitive resins, one from Novabiochem, and the other from Polymer Labs.
A 2-chlorotrityl chloride resin produced according to Example 4 was loaded with Fmoc-L-Leucine, treated with methanol to remove residual reactive chloride and dried. A sample of the resin (1.0 g) was weighed into a 60 mL glass synthesizer vessel with a side port and a removable disk. DCM (10 mL) was charged to the vessel and agitated with nitrogen for 30 minutes, then drained. The leucine derivatized resin is then deprotected by charging 10 mL of a 25% solution of piperidine in N-methylpyrrolidone (NMP), agitating for 10 minutes, draining and repeating once. The deprotection residue was removed by washing with 7x10 mL volumes of NMP. The activated ester of next amino acid in sequence was prepared by dissolving 1.5 eq of amino acid (Fmoc-glu(t-Bu)-OH was the first added in this sequence, see table for charges and formula weights), 1.5 eq of 1-hydroxybenzotriazole (HOBT) (0.149 g) and 1.5 eq of DIEA (0.126 g) into 7.5 mL of NMP at room temperature. The solution was then chilled and 1.5 eq of O-benzotriazol-1-yl-N, N,N',N',-tetramethyluronium hexafluorophosphate (HBTU) (0.370 g) was added and stirred for 30 minutes. DCM (2.5 mL) was then charged to the solution and allowed to stand for 30 minutes. The activated amino acid solution was then charged to the drained resin and agitated with nitrogen. Samples were obtained and analyzed (Kaiser test) each 15 minutes and the results recorded. Upon completion of the reaction the resin was drained and washed with NMP (3x10 mL). This process is then repeated from the deprotection with piperidine for the rest of the amino acids in the sequence. (Glu(tBU), Lys(Boc), Asn(trt), Glu(tBU), Gln(trt), Glu(tBU), leu, leu,).

The results for the three resins are presented below in Table 4, with times expressed as the time to a negative Kaiser Test.

Table 4:

| Peptide Synthesis Efficiency Comparison | | | |
|---|---|---|---|
| Amino Acid | R+H | Polymer Labs | Nova Biochem |
| 1 | 45 | 60 | 60 |
| 2 | 30 | 60 | 60 |
| 3 | 60 | 60 | 60 |
| 4 | 30 | 45 | 45 |
| 5 | 60 | 60 | 60 |
| 6 | 30 | 45 | 45 |
| 7 | 30 | 45 | 30 |
| 8 | 30 | 45 | 30 |
| 9 | 30 | 45 | 45 |
| Total Cycle Time | 345 | 465 | 435 |

The time required for complete reaction with each amino acid added to the growing chain on the bead of this invention is the same or less than for the conventional beads.

Appendix 1 AA usage

| Monomer | fwt | g required |
|---|---|---|
| FMOC Glu (t-Bu) | 425.48 | 0.415 |
| FMOC Lys (Boc) | 468.55 | 0.457 |
| FMOC Asn (trt) | 596.68 | 0.582 |
| FMOC Glu (t-Bu) | 425.48 | 0.415 |
| FMOC Gln (trt) | 610.71 | 0.595 |
| FMOC Glu (t-Bu) | 425.48 | 0.415 |
| FMOC Leu | 353.42 | 0.345 |
| FMOC Leu | 353.42 | 0.345 |
| FMOC Glu (t-Bu) | 425.48 | 0.415 |
| HOBT | 153.15 | 0.149 |
| DIEA | 129.25 | 0.126 |
| HBTU | 379.25 | 0.370 |

| | | | | |
|---|---|---|---|---|
| LeuCT-resin (g)= | 1.00 | | Total NMP | 1035.5 mL |
| Loading Level (mmol/g)= | 0.65 | | Total DCM | 72.5 mL |
| Number of samples= | 1.00 | | Total Piperdine | 32 mL |
| Total resin(g)= | 1.00 | | Total HOBT | 1.344 g |
| Total mmol= | 0.65 | | Total DIEA | 1.134 g |
| Eq. of Monomer Charge= | 1.50 | | Total HBTU | 3.328 g |
| Coupling cycles per step= | 1.00 | | | |
| Monomer usage/Cycle (mmol)= | 0.975 | | | |

| | |
|---|---|
| AA Added | 9 |

Appendix 2 Solvent usage

| | SetUp each sample each cycle | Total each cycle | Total complete |
|---|---|---|---|
| Resin | 1.00g | 1.00g | 1.00g |
| Swell | | | |
| DCM | 10mL | 10 | 10 |
| Stir for 15 min | | | |
| Deprotect | | | |
| 20%Piperidine in NMP | 10mL | 10 | |
| Cycles | 2 | | |
| Total | 20mL | 20 | 160 |
| Stir for 10 min/cycle | | | |
| Wash 1 | | | |
| NMP | 10mL | 10 | |
| Cycles | 7 | | |
| Total | 70mL | 70 | 560 |
| Coupling | | | |
| Monomers See AA Sheet | | | |
| NMP | 7.5mL | 7.5 | 67.5 |
| DCM | 2.5mL | 2.5 | 22.5 |
| Wash 2 | | | |
| NMP | 10 | 10 | |
| Cycles | 3 | | |
| Total | 30 | 30 | 240 |

EP 1 403 290 A1

Final Wash

| | NMP | 10 | 10 | |
|---|---|---|---|---|
| | Cycles | 4 | | |
| | Total NMP | 40 | 40 | 40 |
| | DCM | 10 | 10 | |
| | Cycles | 4 | | |
| | Total DCM | 40 | 40 | 40 |

| | | |
|---|---|---|
| Total NMP | 1035.5mL | |
| Total DCM | 72.5mL | |
| Total Piperdine | 32mL | |

Appendix 3 Kaiser Test Method

The Kaiser test is a test for primary amines and is employed to determine the extent of peptide coupling. A sample of loaded resin (3-20 mg) is placed into a culture tube and evaporated to dryness. The resin is then washed 3 times with ethanol. Five drops of reagent 1 (KCN in pyridine) is added along with 3 drops of reagent 2 (ninhydrin solution) and 3 drops of reagent 3 (phenol in Ethanol). The solution is diluted to 0.5 mL then heated to 75°C for 10 minutes. After 10 minutes the tubes are chilled in a cold water bath. The beads are then observed in front of a white background. A negative test is indicated if the solution is yellow and the beads are transparent. A blue or violet color indicates the presence of free amines and incomplete coupling.

**Claims**

**1.** A crosslinked polymer bead which, when:

(i) functionalized with a 2-chlorotrityl chloride group;
(ii) coupled with Leu to 0.65 mmol/g; and
(iii) coupled with Glu(t-Bu);

allows coupling of FMOC-Lys(BOC)-OH at an amount of 1.5 equivalents in the presence of 1.5 equivalents of HOBT, 1.5 equivalents of DIEA and 1.5 equivalents of HBTU, to be completed, as determined by the Kaiser test, in no more than 35 minutes.

**2.** The bead of claim 1 in which the bead is a styrene polymer.

**3.** A functionalized crosslinked polymer bead produced by a method comprising steps of:

(a) swelling the bead in a first solvent or solvent mixture to a volume from 200% to 310% of its volume when dry; and
(b) contacting the bead with a functionalizing reagent in a second solvent or solvent mixture capable of swelling the bead to a volume from 200% to 310% of its volume when dry.

**4.** The functionalized crosslinked polymer bead of claim 3 in which the bead is a styrene polymer having from 0.5 mole percent to 1.5 mole percent of monomer residues derived from a crosslinker.

**5.** The functionalized crosslinked polymer bead of claim 4 in which the bead is swelled to a volume from 220% to 300% of its volume when dry; and the bead is contacted with a functionalizing reagent in a second solvent or solvent mixture capable of swelling the bead to a volume from 220% to 300% of its volume when dry.

**6.** The functionalized crosslinked polymer bead of claim 5 in which the functionalized bead is loaded with 0.25 to 0.7 meq/g of an amino acid.

**7.** A functionalized crosslinked polymer bead produced by contacting the bead at 100% to 200% of its volume when dry with a functionalizing reagent in a solvent or solvent mixture capable of swelling the bead to a volume from 200% to 400% of its volume when dry.

**8.** The functionalized crosslinked polymer bead of claim 7 in which the polymer bead is at 100% to 150% of its volume when dry when contacted with the functionalizing reagent.

**8.** The functionalized crosslinked polymer bead of claim 7 in which the bead is a styrene polymer having from 0.5 mole percent to 1.5 mole percent of monomer residues derived from a crosslinker.

**9.** The functionalized crosslinked polymer bead of claim 8 in which the solvent or solvent mixture comprises nitrobenzene.

**10.** The functionalized crosslinked polymer bead of claim 9 in which the functionalized bead is loaded with 0.25 to 0.7 meq/g of an amino acid.

**European Patent Office**

## EUROPEAN SEARCH REPORT

Application Number

EP 03 25 5815

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | WO 01 85758 A (LONZA AG) 15 November 2001 (2001-11-15) * the whole document * | 1-10 | C08F8/18 C07K1/04 |
| Y | DE 43 06 839 A (E. BAYER) 8 September 1994 (1994-09-08) * the whole document * | 1-10 | |
| Y | PATENT ABSTRACTS OF JAPAN vol. 1996, no. 12, 26 December 1996 (1996-12-26) & JP 08 217794 A (BIO KOSUMOSU KK), 27 August 1996 (1996-08-27) * abstract * | 1-10 | |
| A | WO 92 22591 A (RESEARCH & DIAGNOSTIC ANTIBODIES) 23 December 1992 (1992-12-23) * claims 1-21 * | 1 | |
| P,X | EP 1 314 745 A (BEADTECH INC.) 28 May 2003 (2003-05-28) * the whole document * | 1-10 | **TECHNICAL FIELDS SEARCHED** (Int.Cl.7) C08F G01N C07K |

The present search report has been drawn up for all claims

| Place of search | Date of completion of the search | Examiner |
|---|---|---|
| THE HAGUE | 2 February 2004 | Permentier, W |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 03 25 5815

This annex lists the patent family members relating to the patent documents cited in the above-mentioned European search report.
The members are as contained in the European Patent Office EDP file on
The European Patent Office is in no way liable for these particulars which are merely given for the purpose of information.

02-02-2004

| Patent document cited in search report | | Publication date | Patent family member(s) | | Publication date |
|---|---|---|---|---|---|
| WO 0185758 | A | 15-11-2001 | AU | 7842401 A | 20-11-2001 |
| | | | WO | 0185758 A2 | 15-11-2001 |
| | | | EP | 1280831 A2 | 05-02-2003 |
| | | | US | 2003092847 A1 | 15-05-2003 |
| DE 4306839 | A | 08-09-1994 | DE | 4306839 A1 | 08-09-1994 |
| JP 08217794 | A | 27-08-1996 | NONE | | |
| WO 9222591 | A | 23-12-1992 | US | 5198531 A | 30-03-1993 |
| | | | AU | 2238992 A | 12-01-1993 |
| | | | DE | 69213401 D1 | 10-10-1996 |
| | | | DE | 69213401 T2 | 27-02-1997 |
| | | | EP | 0543986 A1 | 02-06-1993 |
| | | | JP | 2722392 B2 | 04-03-1998 |
| | | | JP | 6502679 T | 24-03-1994 |
| | | | WO | 9222591 A1 | 23-12-1992 |
| | | | US | 5563220 A | 08-10-1996 |
| EP 1314745 | A | 28-05-2003 | KR | 2003043304 A | 02-06-2003 |
| | | | EP | 1314745 A1 | 28-05-2003 |
| | | | JP | 2003226713 A | 12-08-2003 |
| | | | US | 2003105243 A1 | 05-06-2003 |

For more details about this annex : see Official Journal of the European Patent Office, No. 12/82